(19) Europäisches Patentamt
European Patent Office
Office européen des brevets

(11) EP 0 826 371 A1

(12) EUROPEAN PATENT APPLICATION
published in accordance with Art. 158(3) EPC

(43) Date of publication:
04.03.1998 Bulletin 1998/10

(21) Application number: 96910219.3

(22) Date of filing: 22.04.1996

(51) Int. Cl.[6]: A61K 31/55
// C07D403/04

(86) International application number:
PCT/JP96/01084

(87) International publication number:
WO 96/33722 (31.10.1996 Gazette 1996/48)

(84) Designated Contracting States:
AT BE CH DE DK ES FI FR GB GR IE IT LI LU NL PT SE

(30) Priority: 27.04.1995 JP 129229/95

(71) Applicant: KANEBO LTD.
Sumida-ku, Tokyo 131 (JP)

(72) Inventors:
- TAKAHASHI, Yoshiteru
  Hirakata-shi, Osaka 573-01 (JP)
- HARADA, Shoichi
  Osaka-shi, Osaka 534 (JP)
- INAGI, Toshio
  Mishima-shi, Shizuoka 411 (JP)
- MADA, Akira
  Ihara-gun, Shizuoka 421-33 (JP)
- TAKENO, Satoshi
  Osaka-shi, Osaka 533 (JP)
- SAITO, Tadayuki
  Osaka-shi, Osaka 534 (JP)

(74) Representative:
Hansen, Bernd, Dr. Dipl.-Chem. et al
Hoffmann Eitle,
Patent- und Rechtsanwälte,
Arabellastrasse 4
81925 München (DE)

# (54) EXTERNAL PREPARATION CONTAINING EMEDASTINE

(57) An external preparation which comprises (a) emedastine or a pharmaceutically acceptable salt thereof and (b) one or more bases selected from the group consisting of liquid paraffin, light liquid paraffin and gelled hydrocarbons. The preparation is chemically stable and suffers from little color change and no decrease in the content of the active ingredient during storage. Also, it suffers from little morphological change and remains physically stable, thus showing a high storage stability. When applied, the preparation exerts an antihistaminic effect. It causes scarcely any skin irritation and thus shows a high safety. Accordingly, the preparation is useful in the topical treatment of allergic diseases, in particular, urticaria, eczema/dermatitis, pruritus cutaneus, prurigo, etc.



Printed by Xerox (UK) Business Services
2.15.11/3.4

## Description

FIELD OF ART

This invention relates to external preparations comprising emedastine represented by the formula

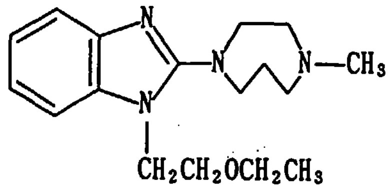

or its pharmaceutically acceptable salt and one or more bases selected from liquid paraffin, light liquid paraffin and hydrocarbon gel.

The external preparations of this invention can be useful in the topical treatment of allergic skin diseases, in particular, urticaria, eczema/dermatitis, pruritus cutaneus, prurigo, etc.

BACKGROUND OF INVENTION

Emedastine and its pharmaceutical acceptable salt used as an active ingredient in this invention are known (Japanese Patent Publication No.24821/1990, US Patent No.4,430,343), and it is also known that they have an activity of restraining the release of histamine and antihistaminic activity [Fukuda et al., Arzneimittel-Forshung Drug Research, 34(II), 7,805(1984)], and that they are useful for prophylaxis and the treatment of diseases caused by histamine, e.g. allergic dermatosis and allergic rhinitis.

The preparation (capsule) for oral administration containing emedastine difumarate as an active ingredient has been approved as a drug for the treatment of allergic rhinitis and urticaria by the Japanese Minister of Health and Welfare.

As regards the external preparations containing emedastine or its pharmaceutically acceptable salt, it is disclosed in the above mentioned US Patent (US Patent No.4,430,343) that the ointment can be prepared by using a conventional base such as petrolatum and polyethylene glycol, and in Example of the above mentioned US Patent, there is disclosed an ointment comprising emedastine difumarate and polyethylene glycol. And in Japanese Patent Publication (without examination) [No.83924/1991 corresponded to EP 440811], the compositions for percutaneous administration comprising emedastine and an aliphatic monocarbonic acid lower alkyl ester or an aliphatic dicarbonic acid dilower alkyl ester is disclosed. But the above mentioned compositions are ones for percutaneous administration having excellent percutaneous absorption ability of emedastine, that is, ones for percutaneous administration of emedastine based on the systemic action.

On the other hand, the ointment disclosed in the above mentioned US Patent is suitable for an external preparation for the topical application, but it is found to tend to be strongly stained on the storage condition.

DISCLOSURE OF INVENTION

The object of this invention is to provide the external preparations containing emedastine or its pharmaceutically acceptable salt which are suitable for the topical application and are superior in the stability in storage.

The present inventors have extensively engaged in the bases used for the external preparations to attain the above object, and found that the external preparations prepared by dissolving or suspending emedastine or its pharmaceutically acceptable salt in one or more bases selected from liquid paraffin, light liquid paraffin and hydrocarbon gel are superior in the stability in storage, and that accomplished this invention.

BEST MODE FOR PRACTICING INVENTION

The external preparations of this invention can be ones prepared by dissolving or suspending emedastine or its pharmaceutically acceptable salt in one or more bases selected from liquid paraffin, light liquid paraffin and hydrocarbon gel. In addition, if necessary, to the said preparations can be added the conventional additives used in the pharmaceutical or cosmetic preparations: an emulsifying agent (e.g. a surfactant, such as polyoxyethylene cetyl ether, polyoxyethylene sorbitan monostearate, etc.), a preservative (e.g. propyl parahydoxybenzoate, butyl parahydroxybenzoate, etc.), an antioxidant (e.g. sodium bisulfite, disodium edetate, etc.), a viscosity-regulator (e.g. carboxyvinyl polymer, acacia, xanthan gum, sodium alginate, carmellose sodium, etc.), a buffer agent (e.g. monobasic potassium phosphate, dibasic sodium phosphate, etc.), a solvent (e.g. water, ethanol, etc.), a pH-controlling agent (e.g. sodium hydroxide, potassium hydroxide, etc.), or other additives (e.g. olive oil, peanut oil, cotton seed oil, etc.).

The external preparations of this invention include ointments, lotions, o/w type creams, w/o type creams, etc., among which oily ointments and o/w type creams are preferably useful.

The pharmaceutically acceptable salts of emedastine include its monofumarate, its difumarate, its phosphate, its borate, its hydrochloride, its sulfate, its citrate, its maleate, etc.

The amount of emedastine or its pharmaceutically acceptable salt contained in the external preparation of this invention is preferably 0.01 - 10% by weight as emedastine difumarate, more preferably 0.1 - 5% by weight.

Liquid paraffin or light liquid paraffin used in this invention is a mixture of liquid hydrocarbons prepared from petroleum, preferably one described on page 1016 or 1017 of the Pharmacopeia of Japan 12th edition (Daiichi Houki Syuppan Co., Ltd. 1991, abbreviated to JP hereinafter). Liquid paraffin is available in the trade names, SILKOOL P-350P, MORESCO WHITE P350P, MOLESCO VIOLESS U-8 (manufactured by Matsumura Oil Research Corp.) and Kaydol (manufactured by Witco Sonneborn Division Inc.). Light liquid paraffin is available in the trade names, SILKOOL P-70, MORESCO WHITE P70 (manufactured by Matsumura Oil Research Corp.) and CARNATION (manufactured by Witco Sonneborn Division Inc.).

Hydrocarbon gel used in this invention is one prepared by gelatinizing liquid paraffin conformed to the standard of JP, with polyethylene in amount of 5 - 10% of the paraffin. Ones described on page 156 of the Standard of Japanese Pharmaceutical Excipients (Yakuji Nippo Co., Ltd. 1993) are preferably used. These are available in the trade names, POLOID (manufactured by Maruishi Pharm. Co., Ltd.) and PLASTIBASE (manufactured by Bristol-Myers Squibb Inc.).

The amount of the said liquid paraffin, light liquid paraffin and hydrocarbon gel respectively depends upon the preparation forms, and therefore is suitably adjusted according to the physicochemical property, the viscosity, etc. necessary for the objected external preparations.

The external preparations of this invention can be commonly prepared by adding emedastine or its pharmaceutically acceptable salt to one or more bases selected from liquid paraffin, light liquid paraffin and hydrocarbon gel, and to the said preparations, if necessary, by adding a higher fatty acid and a higher alcohol, an emulsifying agent such as a surfactant, a preservative, an antioxidant, a viscosity-regulator, a solvent, a buffer agent, a pH-controlling agent or other additives which are commonly used in the preparation of the pharmaceutical preparation or the cosmetic preparation mentioned above.

An oily ointment among the external preparations of this invention is prepared as follows:
Liquid paraffin or light liquid paraffin and hydrocarbon gel are mixed and stirred, and then to the mixture is added emedastine or its pharmaceutically acceptable salt, and if necessary, a higher fatty acid, a higher alcohol, a preservative and/or an antioxidant, and the resulting mixture is uniformly suspended to prepare the external preparation (oily ointment) of this invention.

The total amount of liquid paraffin and light liquid paraffin is preferably 1 to 40% by weight per total weight of the oily ointment.

The amount of hydrocarbon gel is usually 60 to 99% by weight per total weight of the oily ointment.

A higher fatty acid and/or a higher alcohol or other additives are added according to the physicochemical property, viscosity, etc. of the oily ointment, if necessary.

As higher fatty acids, there are mentioned myristic acid, palmitic acid, stearic acid, etc.

As higher alcohols, there are mentioned myristyl alcohol, cetanol, cetostearyl alcohol, stearyl alcohol, 2-octyldodecanol, behenyl alcohol, etc.

As other additives, there are mentioned olive oil, peanut oil, cotton seed oil, etc.

As preservatives, there are mentioned methyl parahydoxybenzoate, ethyl parahydroxybenzoate, propyl parahydroxybenzoate, butyl parahydroxybenzoate, etc. The above mentioned preservative can be used alone, but a mixture of two or more thereof is preferably used.

As antioxidants, there are mentioned tocopherol acetate, tocopherol, butylhydroxyanisol, etc.

To the oily ointment of this invention, if necessary, such bases as solid paraffin, purified lanolin, white petrolatum, etc. can be added other than liquid paraffin, light liquid paraffin and hydrocarbon gel as far as they do not interfere with the effect of this invention.

An o/w type cream among the external preparations is prepared by follows:
Liquid paraffin or light liquid paraffin as the oil phase, and if necessary, hydrocarbon gel, and furthermore, if necessary, one or more additives selected from a higher fatty acid, a higher alcohol and a nonionic surfactant, and a preservative, an antioxidant and other additives are stirred in the homogenizer at 70 - 80°C to be dissolved. On the other hand, as the aqueous phase, emedastine difumarate is dissolved in water, and if necessary, a preservative, an antioxidant, a pH-controlling agent, and a viscosity-regulator can be added to the solution, and then the solution is warmed to 70 - 80° C. Thus the aqueous phase prepared, is gradually added to the oil phase prepared under stirring. The mixture thereof is

kept under stirring for more than 10 minutes at 3000 - 5000 rpm, and then the mixture is cooled to room temperature under stirring to prepare an o/w type cream by inverting the phase. And furthermore, if necessary, an antioxidant is uniformly admixed to the above mentioned cream to prepare the preparation (o/w type cream) of this invention.

An o/w type cream is also prepared as follows:
As the oil phase, liquid paraffin or light liquid paraffin, and if necessary, hydrocarbon gel, and furthermore, if necessary, one or more additives selected from a higher fatty acid, a higher alcohol and a nonionic surfactant, and a preservative, an antioxidant and other additives are stirred in the homogenizer, and the mixture thereof is warmed to 70 - 80°C to be dissolved, and then emedastine difumarate is suspended in it under stirring. On the other hand, as the aqueous phase, water and if necessary, a preservative, an antioxidant, a pH-controlling agent, and a viscosity-regulator are mixed and the mixture thereof is warmed to 70 - 80°C. The aqueous phase, then is gradually added to the oil phase under stirring and the mixture thereof is kept under stirring at 3000 - 5000rpm for more than 10 minutes. Then the mixture is cooled to room temperature under stirring to prepare an o/w type cream by inverting the phase. Furthermore, if necessary, an antioxidant is uniformly admixed to the above mentioned cream to prepare the preparation (w/o type cream) of this invention.

The total amount of liquid paraffin and light liquid paraffin is usually preferably 1 - 50% by weight per the total weight of the cream, more preferably 10 - 30% by weight.

Hydrocarbon gel is, if necessary, added according to the physicochemical property, viscosity, etc. of the cream. The amount of hydrocarbon gel is usually preferably 1 - 50% by weight per the total weight of the cream.

A higher fatty acid and/or a higher alcohol are, if necessary, added according to the physicochemical property, viscosity, etc. of the cream.

As higher fatty acids, the above mentioned ones are used, and especially stearic acid, which is described on page 949 in JP, is preferably used and available in a trade name, NAA-175 (NOF Corp.). The amount of the said higher fatty acid is usually preferably 0 - 10% by weight per the total weight of the cream.

As higher alcohols, the above mentioned ones are used, especially cetostearyl alcohol, stearyl alcohol and cetanol are preferably used and these are available in the trade name, NNA-48, NNA-45, NNA-44 (NOF Corp.) respectively. The amount of the said higher alcohol is usually preferably 0 - 20% by weight per the total weight of the cream.

A nonionic surfactant used in the cream of this invention is not particularly limited in kind and is preferably one at an HLB of 10 or more than 10.

As nonionic surfactants at an HLB of 10 or more than 10, there are mentioned polyoxyethylene sorbitan monooleate to which 6 - 20 moles of ethylene oxide are added, polyoxyethylene sorbitan trioleate to which 20 moles of ethylene oxide are added, polyoxyethylene sorbitan monolaurate to which 20 moles of ethylene oxide are added, polyoxyethylene sorbitan monopalmitate to which 20 moles of ethylene oxide are added, polyoxyethylene sorbitan monostearate to which 20 moles of ethylene oxide are added, polyoxyethylene sorbitan tristearate to which 20 moles of ethylene oxide are added, polyoxyethylene sorbitan monoisostearate to which 20 moles of ethylene oxide are added, polyoxyethylene monolaurate to which 10 moles of ethylene oxide are added, polyoxyethylene monostearate to which 10 - 55 moles of ethylene oxide are added, polyoxyethylene monooleate to which 10 moles of ethylene oxide are added, polyoxyethylene lauryl ether to which 4 - 25 moles of ethylene oxide are added, polyoxyethylene cetyl ether to which 5 - 40 moles of ethylene oxide are added, polyoxyethylene stearyl ether to which 20 moles of ethylene oxide are added, polyoxyethylene oleyl ether to which 7 - 50 moles of ethylene oxide are added, polyoxyethylene behenyl ether to which 10 - 30 moles of ethylene oxide are added, polyoxyethylene nonylphenyl ether to which 7 - 20 moles of ethylene oxide are added, polyoxyethylene octylphenyl ether to which 10 - 30 moles of ethylene oxide are added, polyoxyethylene hydrogenated castor oil to which 20 - 100 moles of ethylene oxide are added, polyoxyethylene castor oil to which 20 - 60 moles of ethylene oxide are added, polyoxyethylene sorbitol tetraoleate to which 30 - 60 moles of ethylene oxide are added, and so on.

As the preferred nonionic surfactants among them, polyoxyethylene cetyl ether to which 15 or 20 moles of ethylene oxide are added, polyoxyethylene sorbitan monostearate to which 20 moles of ethylene oxide are added, and polyoxyethylene hydrogenated castor oil to which 50 moles of ethylene oxide are added can be mentioned. These are available respectively in the trade name, NIKKOL BC-15TX, NIKKOL BC-20TX, NIKKOL TS-10 , NIKKOL HCO-50 (manufactured by Nikko Chemicals Co., Ltd.).

The above mentioned nonionic surfactant can be used either alone or, if necessary, in the form of a mixture of two or more ones. On the other hand, the nonionic surfactant at an HLB of 10 or more than 10 mentioned above and a nonionic surfactant at an HLB of less than 10 mentioned below can be used in the form of a mixture. In this case, HLB value of the mixture is preferably 10 or more than 10.

As nonionic surfactants at an HLB of less than 10 mentioned above, there are mentioned sorbitan monocaprylate, sorbitan monolaulate, sorbitan monopalmitate, sorbitan monostearate, sorbitan sesquistearate, sorbitan tristearate, sorbitan monooleate, sorbitan sesquioleate, sorbitan trioleate, sorbitan monoisostearate, sorbitan sesquiisostearate, glyceryl monocaprylate, glyceryl monomyristate, glyceryl monostearate, glyceryl monooleate, glyceryl monoisostearate, polyoxyethylene monostearate to which 1 to 4 moles of ethyleneoxide is added, polyoxyethylene monooleate to

which 2 to 6 moles of ethyleneoxide is added, polyoxyethylene lauryl ether to which 2 moles of ethylene oxide are added, polyoxyethylene cetyl ether to which 2 moles of ethylene oxide are added, polyoxyethylene stearyl ether to which 2 - 4 moles of ethylene oxide are added, polyoxyethylene oleyl ether to which 2 moles of ethylene oxide are added, polyoxyethylene behenyl ether to which 5 moles of ethylene oxide are added, polyoxyethylene nonylphenyl ether to which 2 - 5 moles of ethylene oxide are added, polyoxyethylene octylphenyl ether to which 3 moles of ethylene oxide are added, polyoxyethylene hydrogenated castor oil to which 5 - 10 moles of ethylene oxide are added, polyoxyethylene castor oil to which 3 -10 moles of ethylene oxide are added, propylene glycol monostearate and so on.

The amount of the said nonionic surfactant is usually preferably 0.5 - 10% by weight per the total weight of the cream.

As antioxidants used in the cream, there are mentioned sodium bisulfite, ascorbic acid, disodium edetate, etc. other than ones mentioned above. The amount of the said antioxidant is usually preferably 0.01 - 10% by weight per the total weight of the cream.

As pH-controlling agents, there are mentioned sodium hydroxide, potassium hydroxide, diisopropanolamine, etc. The amount of the said pH-controlling agent is usually preferably 0.01 - 10% by weight per total weight of the cream.

As preservatives and other additives used in the o/w type cream, there are used the same ones as mentioned above.

To the o/w type cream of this invention, if necessary, other bases (e.g. solid paraffin, purified lanolin, white petrolatum, etc.) than liquid paraffin, light liquid paraffin and hydrocarbon gel are added as far as they do not interfere with the effect of this invention.

As viscosity-regulators, there are mentioned carboxyvinyl polymer, acacia, xanthan gum, sodium alginate, carmellose sodium, methylcellulose, hydroxypropylcellulose, carboxymethylcellulose, polyvinylpyrrolidone, polyvinylalcohol, etc. The amount of the said viscosity-regulator is usually preferably 0.1-2% by weight per the total weight of the cream.

A lotion among the external preparations of this invention is prepared as follows:

As the oil phase, liquid paraffin or light liquid paraffin, and if necessary, hydrocarbon gel, and furthermore, if necessary, one or more additives selected from a higher fatty acid, a higher alcohol and a nonionic surfactant, and an antioxidant are mixed and stirred in the homogenizer at 70 - 80°C to be dissolved. On the other hand, as the aqueous phase, emedastine or its pharmaceutically acceptable salt and a surfactant, and if necessary, an antioxidant, a preservative, a viscosity-regulator agent and a pH-controlling agent are dissolved in water previously warmed. The aqueous phase thus prepared is gradually added to the oil phase under stirring, and the said mixture is stirred for more than 10 minutes at 3000 - 5000rpm. The mixture is cooled to room temperature by adding water, and if necessary, glycerin. If necessary, an antioxidant is admixed uniformly to the said mixture to prepare the external preparation (lotion) of this invention.

A lotion is also prepared as follows:

As the oil phase, liquid paraffin or light liquid paraffin, and if necessary, hydrocarbon gel, and furthermore, if necessary, one or more additives selected from a higher fatty acid, a higher alcohol and a nonionic surfactant, and an antioxidant are mixed and stirred in the homogenizer at 70 - 80°C to be dissolved, and then emedastine difumarate is suspended in it under stirring. On the other hand, as the aqueous phase, water, and if necessary, an antioxidant, a preservative, a viscosity-regulator and a pH-controlling agent are dissolved in water. To the oil phase is gradually added the said aqueous phase under stirring, and the mixture is stirred for more than 10 minutes at 3000 - 5000rpm, and then water, and if necessary, glycerin is added to it and the mixture is cooled to room temperature under stirring, and furthermore, if necessary, an antioxidant is uniformly admixed to the said mixture to prepare the external preparation (lotion) of this invention.

The total amount of liquid paraffin and the light liquid paraffin is usually preferably 0.1 - 50% by weight per the total weight of the lotion.

If necessary, a higher fatty acid and/or a higher alcohol or other additives can be added according to the physicochemical property, viscosity, etc. of the lotion.

A higher fatty acid, a higher alcohol, an antioxidant, a preservative, a viscosity-regulator, a pH-controlling agent and other additives used in the lotion are the same ones as mentioned above.

As surfactants, there are used the same ones as the nonionic surfactants as mentioned above.

pH of the external preparation of this invention is preferably 3 - 9, more preferably 5 - 7 in order to avoid the epidermal irritation etc.

The external preparation of this invention can be useful for the topical treatment of allergic diseases, in particular, urticaria, eczema/dermatitis, pruritus cutaneus, prurigo, etc.

The dosage of the external preparation of this invention varies depending on age, weight, disease, etc. of a patient, but it is usually administered in the dosage of 0.1 - 100mg as emedastine per once for an adult and the dosage is applied on the skin once or several times per day.

The external preparation of this invention is chemically and physically stable, as the change in the color is less and the content of the active ingredient does not decrease and the morphological change is less on the storage. Therefore, the preparation is superior in the stability in storage (see Tests No.1 - 3). And by applying the external preparation of

this invention, it exhibits the strong antihistamic activity on the applied region(see Test 4) and it hardly exhibits the activity on the other region. Therefore, the external preparation of this invention is suitable for the topical treatment. In addition, the external preparation is safe for almost no exhibition of any epidermal irritation.

As mentioned above, the external preparation of this invention can be useful for the topical treatment of allergic diseases, in particular, urticaria, eczema/dermatitis, pruritins cutaneus, prurigo, etc.

This invention is described in further detail by illustrating test examples.

[Test example]

Test 1 (stability in base)

1. Preparation of sample

Emedastine difumarate (1.0g) is uniformly suspended in a base (light liquid paraffin, liquid paraffin or hydrocarbon gel) (19g) to prepare the preparations of this invention (No.1 - 3).

On the other hand, emedastine difumarate (1.0g) is uniformly suspended or dissolved in a base (white petrolatum, microcrystalline wax, propylene glycol, MACROGOL 400, solid paraffin, white beeswax, caster oil or purified lanolin) (19.0g) other than the above base to prepare comparative preparations (No.1 - 8).

2. Test method

The color, right after preparing a sample, was measured by colorimetry (Σ 80 COLOR MEASURING SYSTEM, NIPPON DENSHOKU IND. Co., Ltd.). Then each sample was put in a glass bottle, and the bottle was kept at 60°C with a tight stopper. After a week and two weeks, the color was respectively measured by using the colorimetry, and the change in standing of the color was evaluated.

3. Test result

The color difference (NBS unit) was shown in Table 1.

Table 1

| Sample | Base | Term of storage | | |
|---|---|---|---|---|
| | | right after | 1 week | 2 week |
| external preparation of the invention (1) | light liquid paraffin | 0 | 0.5 | 0.4 |
| external preparation of the invention (2) | liquid paraffin | 0 | 0.5 | 0.8 |
| external preparation of the invention (3) | hydrocarbon gel | 0 | 0.4 | 0.6 |
| comparative preparation (1) | white petrolatum | 0 | 1.8 | 4.5 |
| comparative preparation (2) | microcrystalline wax | 0 | 2.5 | 3.8 |
| comparative preparation (3) | propylene glycol | 0 | 8.6 | 10.5 |
| comparative preparation (4) | MACROGOL 400 | 0 | 7.4 | 7.9 |
| comparative preparation (5) | solid paraffin | 0 | 1.4 | 2.1 |
| comparative preparation (6) | white bees wax | 0 | 3.2 | 3.6 |
| comparative preparation (7) | caster oil | 0 | 1.3 | 2.3 |
| comparative preparation (8) | purified lanolin | 0 | 2.1 | 2.4 |

As is clear from Table 1, the external preparations of this invention prepared by using light liquid paraffin, liquid paraffin or hydrocarbon gel showed less the change in color. On the other hand, all the comparative samples prepared by using a base other than the above bases were stained.

Test 2 (stability test in storage)

1. Sample

(a) external preparation of Example 1 (oily ointment)
(b) external preparation of Example 2 (o/w type cream)
(c) external preparation of Example 3 (o/w type cream)
(d) external preparation of Example 4 (o/w type cream)
(e) external preparation of Comparative example 1 (polyethylene glycol ointment)

2. Test method

The color of the sample, right after preparing, was measured by the colorimetry (Σ 80 COLOR MEASURING SYSTEM, NIPPON DENSHOKU IND. Co., Ltd.). Then each sample was put in a bottle and the bottle was kept at 40°C with a tight stopper. After 2 months, the color was measured in the same method as mentioned above, and the change in standing of the color was evaluated.

3. Test result

The color difference (NBS unit) was shown in Table 2.

Table 2

| Sample | Term of storage | |
|---|---|---|
| | right after | 2 months |
| external preparation of Example 1 | 0 | 0.5 |
| external preparation of Example 2 | 0 | 0.8 |
| external preparation of Example 3 | 0 | 0.8 |
| external preparation of Comparative example 1 | 0 | 14.7 |

As is clear from Table 2, the preparations of this invention showed less the change in color. On the other hand, the comparative sample was stained. The external preparation of Example 4 was not stained.

Test 3 (stability test in storage)

1. Sample

(a) external preparation of Example 1 (oily ointment)
(b) external preparation of Example 2 (o/w type cream)
(c) external preparation of Example 3 (o/w type cream)
(d) external preparation of Example 4 (o/w type cream)
(e) external preparation of comparative example 1 (polyethylene glycol ointment)

2. Test method

The sample was put in a bottle and the bottle was kept for 2 months with a tight stopper. After the storage, the amount of emedastine difumarate in the sample was determined by liquid chromatography (HPLC) using paramethylbenzophenone as an internal standard substance under the following condition.

The percent of remain was calculated based on the difference between the content of the drug just before the test and the content of the drug after the storage.

Condition of HLPC

Column: Inertsil ODS-3 (150mm X 4.6mm, GL Sciences Inc.)
Mobile phase: A mixed solution of one part of the solution prepared by dissolving sodium dihydrogen phosphate

dihydrate (3.9g), sodium laurylsulfate (2.5g) in purified water (1000ml), adjusted pH to 2.4 by phosphoric acid, and one part of acetonitrile.

Column temperature: 40°C

Flow rate: 1.2ml/min.

Detection method: absorbance at UV 280nm.

3. Test result

The result was shown in the Table 3.

Table 3

| Sample | Percent of remain after 2 months storage at 40°C |
|---|---|
| external preparation of Example 1 | 99.1 |
| external preparation of Example 2 | 99.9 |
| external preparation of Example 3 | 99.6 |
| external preparation of Example 4 | 99.3 |
| external preparation of Comparative example 1 | 94.3 |

As is clear from the Table 3, the external preparations of this invention did not cause the decrease of the amount of emedastine and the said preparations were chemically stable. And the external preparations of this invention did not cause the morphological change by the storage and the said preparations ware physically stable.

Test 4 (Pharmacological test)

The efficacy of the external preparations of this invention was evaluated based on the increase of vascular permeability induced by histamine in rats.

1. Sample

(a) external preparation of Example 1 (oily ointment)
(b) external preparation of Example 2 (o/w type cream)

2. Test method

A sample (50μl) was applied at the two parts on the back of a Wistar male rat (8 weeks, 164 - 198g, Japan SLC), and the applied parts were covered by aluminum foil (2.5 X 5cm), and the parts were fixed by the adhesive and elastic bandage (Benefix No.3, 5X25cm, Nippon Sigmax Co., Ltd.). Three hours after the application, dye (0.5% Evans blue) was intravenously administered to rats (1ml/rat), and right after, histamine (200μg/ml as free base) was intracutaneously injected into the parts where the sample was applied, and on the other hand, physiological saline (50μl) was intracutaneously injected into the two parts on the back but different from the parts applied the sample. Thirty minutes later, the rat was killed, and two parts of the skin where histamine was injected and two parts of the skin where saline was injected were gathered respectively, and according to the Katayama's method [(Microbiol. Immunol., 22, 89-101(1978)], the dye was extracted from the skin where the dye was leaked out. The absorbance at 620nm was measured and the amount of the leaked dye was determined by using the calibration curve. On the other hand, to a control group, the external preparations in which emedastine difumarate was not contained (prepared in accordance with Example 1 or 2 except for containing no emedastine difumarate) were applied in stead of the external preparation of Example 1 or 2. The test was conducted in the same way as mentioned above, and the amount of the leaked dye was determined. The percent of the inhibition of the sample-applied group against control group (mean of 10 rats) was calculated on following formula;

$$\text{Inhibition (\%)} = [(C-Bc)-(D-Bd)]/(C-Bc)X100$$

D: sample applied group; amount of dye leaked at histamine-injected region

C: control group; amount of dye leaked at histamine-injected region

Bd: sample applied group; amount of dye leaked at saline-injected region

Bc: control group; amount of dye leaked at saline-injected region

3. Test result

The percent of inhibition (mean ± S.E.) was shown in Table 4.

Table 4

| Sample | Inhibition rate (%) |
|---|---|
| Example 1 | 76.6±4.3 |
| Example 2 | 91.3±1.1 |

As is clear from Table 4, the external preparations of this invention showed the strong inhibitory activity on the increase of vascular permeability induced by histamine.

This invention is explained in further detail by illustrating Examples and a Comparative example.

Example 1 (oily ointment)

Emedastine difumarate (1.0g) and butyl parahydoxybenzoate (0.1g) were suspended in light liquid paraffin (trade name: SILKOOL P-70, Matsumura Oil Research Corp.) (5.0g) and the mixture was crushed in the mortar, filtered by 200 mesh sieve. The filtrate and liquid paraffin (5.0g) were mixed and the mixture was added to hydrocarbon gel (trade name: POLOID, Maruishi Pharm. Co., Ltd.)(88.9g) which was warmed to about 60°C to be uniformly dispersed. Thus the external preparation (oily ointment) was prepared.

Example 2 (o/w type cream)

As an oily phase, a mixture of 81.0g of light liquid paraffin (trade name: SILKOOL P-70, Matsumura Oil Research Corp.), 3.0g of stearic acid (trade name: NAA-175, NOF Corp. ), 27.0g of cetostearyl alcohol (trade name: NAA-48, NOF Corp.), 15.0g of polyoxyethylene cetyl ether (trade name: NIKKOL BC-15TX, Nikko Chemicals Co., Ltd.) and 0.3g of butyl parahydroxybenzoate was stirred in the homogenizer at about 80°C to be dissolved. On the other hand, as an aqueous phase, 3.0g of emedastine difumarate and 0.3g of methyl parahydroxybenzoate were dissolved in 159.3g of purified water and to the solution was added 11.1g of an 10 %w/w aqueous potassium hydroxide solution as a pH-controlling agent and the mixture was warmed to 80°C. The aqueous phase was gradually added to the oil phase under stirring and the said mixture was stirred for 10 minutes at about 4000rpm, and then cooled to room temperature under stirring to prepare the external preparation (o/w type cream) of this invention by inverting the phase into o/w type cream.

Example 3 (o/w type cream)

As an oil phase, a mixture of 43.5g of light liquid paraffin (trade name: CARNATION, Witco Sonneborn Division ), 3.0g of stearic acid (trade name: NAA-175, NOF Corp.), 15.0g of cetostearyl alcohol (trade name: NAA-48, NOF Corp.), 8.1g of polyoxyethylene cetyl ether (trade name: NIKKOL BC-20TX, Nikko Chemicals Co., Ltd.) and 0.3g of butyl parahydroxybenzoate was stirred in the homogenizer at about 80°C to be dissolved. On the other hand, as an aqueous phase, 3.0g of emedastine difumarate and 0.3g of methyl parahydoxybenzoate were dissolved in 225.72g of purified water and to the solution was added 1.08g of potassium hydroxide as a pH-controlling agent, and the said mixture was warmed to 80°C. The aqueous phase was gradually added to the oil phase under stirring and the said mixture was stirred for 10 minutes at about 4000rpm, and then cooled to room temperature under stirring to prepare the external preparation (o/w type cream) of this invention.

Example 4 (o/w type cream)

As an oil phase, a mixture of 18.0g of light liquid paraffin (trade name: CARNATION, Witco Sonneborn Division ), 5.0g of white petrolatum (WHITE PROTOPET 1S, Witco Sonneborn Division), 2.0g of cetostearyl alcohol (trade name: NAA-48, NOF Corp.), 0.4g of polyoxyethylene sorbitan monostearate (trade name: NIKKOL TS-10, Nikko Chemicals Co., Ltd.) and 0.3g of polyoxyethylene hydrogenated castor oil 50 (trade name: NIKKOL HCO-50, Nikko Chemicals Co.,

Ltd.) were stirred in the homogenizer at about 80°C to be dissolved. In the solution was dispersed 1.0g of emedastine difumarate under stirring. On the other hand, as an aqueous phase, to 72.4g of purified water was added 0.5g of carboxyvinyl polymer (trade name: HIVISWAKO-104, Wako Pure Chemical Ind. Ltd.) to swell, and then to it was added 0.3g of sodium hydroxide and 0.1g of sodium hydrogen sulfite to be dissolved, and the solution was warmed to about 80°C. The aqueous phase was gradually added to the oil phase under stirring and was cooled to room temperature under stirring in the homogenizer to prepare the external preparation (o/w type cream) of this invention.

Comparative example 1 (polyethylene glycol ointment)

A mixture of 148.5g of polyethylene glycol 400 (trade name: MCROGOL 400, Sanyo Chemical Ind. Ltd.) and 148.5g of polyethyleneglycol 4000 (trade name: MACROGOL 4000, Sanyo Chemical Ind. Ltd.) was warmed to 60°C to be dissolved. To this solution was added 3.0g of emedastine difumarate to be dissolved and the solution was cooled to room temperature under stirring to prepare the external preparation (polyethylene glycol ointment) of Comparative example 1.

## Claims

1. An external preparation comprising (a) emedastine or its pharmaceutically acceptable salt and (b) one or more bases selected from liquid paraffin, light liquid paraffin and hydrocarbon gel.

2. An external preparation comprising (a) emedastine or its pharmaceutically acceptable salt, (b) one or more bases selected from liquid paraffin, light liquid paraffin and hydrocarbon gel, and (c) one or more additives selected from a higher fatty acid, a higher alcohol, a nonionic surfactant and water.

3. The external preparation claimed in claim 1 or 2 in which the base is a combination of liquid paraffin or light liquid paraffin and hydrocarbon gel.

4. The external preparation claimed in any of claims 1 to 3 in which the preparation is an oily ointment.

5. The external preparation claimed in any of claims 1 to 3 in which the preparation is an o/w type cream.

6. The external preparation claimed in any of claims 1 to 5 in which a pharmaceutically acceptable salt of emedastine is emedastine difumarate.

7. An oily ointment comprising (a) emedastine difumarate, ($b_1$) liquid paraffin or light liquid paraffin, and ($b_2$) hydrocarbon gel.

8. An o/w type cream comprising (a) emedastine difumarate, (b) liquid paraffin or light liquid paraffin, ($c_1$) a higher fatty acid and /or a higher alcohol, ($c_2$) a nonionic surfactant, and ($c_3$) water.

9. An o/w type cream comprising (a) emedastine difumarate, (b) light liquid paraffin, ($c_1$) stearic acid and/or cetostearyl alcohol, ($c_2$) polyoxyethylene cetyl ether, and ($C_3$) water.

10. An o/w type cream comprising (a) emedastine difumarate, (b) liquid paraffin or light liquid paraffin, ($c_1$) a higher fatty acid and/or a higher alcohol, ($c_2$) a nonionic surfactant, ($c_3$) an antioxidant, and ($c_4$) water.

11. An o/w type cream comprising (a) emedastine difumarate, (b) light liquid paraffin, ($c_1$) stearyl alcohol and/or cetanol, ($c_2$) one or more nonionic surfactants, ($c_3$) sodium bisulfite, and ($c_4$) water.

## INTERNATIONAL SEARCH REPORT

International application No.
PCT/JP96/01084

**A. CLASSIFICATION OF SUBJECT MATTER**

Int. $Cl^6$ A61K31/55 // C07D403/04

According to International Patent Classification (IPC) or to both national classification and IPC

**B. FIELDS SEARCHED**

Minimum documentation searched (classification system followed by classification symbols)

Int. $Cl^6$ A61K31/55 // C07D403/04

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| Y | JP, 7-33665, A (Kanebo, Ltd.), February 3, 1995 (03. 02. 95), Full descriptions (Family: none) | 1 - 11 |
| Y | JP, 3-83924, A (Kanebo, Ltd.), April 9, 1991 (09. 04. 91), Full descriptions & WO, 91/3244, A & EP, 440811, A & US, 5321022, A | 1 - 11 |
| Y | JP, 7-97326, A (Hisamitsu Pharmaceutical Co., Inc.), April 11, 1995 (11. 04. 95), Full descriptions (Family: none) | 1 - 11 |
| Y | WO, 95/4551, A1 (Fujisawa Pharmaceutical Co., Ltd.), February 16, 1995 (16. 02. 95), Full descriptions & JP, 7-506333, A | 1 - 11 |
| Y | JP, 6-65596, A (Shiseido Co., Ltd.), March 8, 1994 (08. 03. 94), | 1 - 11 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| July 11, 1996 (11. 07. 96) | July 23, 1996 (23. 07. 96) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP96/01084

C (Continuation). DOCUMENTS CONSIDERED TO BE RELEVANT

| Category* | Citation of document, with indication, where appropriate, of the relevant passages | Relevant to claim No. |
|---|---|---|
| | Full descriptions & WO, 91/19562, A & EP, 500941, A1 & AU, 9179617, A | |
| Y | JP, 6-345637, A (Yugen Kaisha Nonokawa Shoji), December 20, 1994 (20. 12. 94), Full descriptions (Family: none) | 1 - 11 |
| Y | JP, 5-246892, A (Pola Chemical Industry Inc.), September 24, 1993 (24. 09. 93), Full descriptions (Family: none) | 1 - 11 |
| Y | JP, 4-243827, A (Sumitomo Pharmaceuticals Co., Ltd.), August 31, 1992 (31. 08. 92), Full descriptions (Family: none) | 1 - 11 |
| Y | JP, 3-118323, A (Tokyo Tanabe Co., Ltd.), May 20, 1991 (20. 05. 91), Full descriptions (Family: none) | 1 - 11 |
| Y | JP, 3-24015, A (S.S. Pharmaceutical Co., Ltd.), February 1, 1991 (01. 02. 91), Full descriptions & EP, 473811, A & US, 5112816, A & CA, 2024612, A | 1 - 11 |
| Y | JP, 63-5022, A (A. Nattermann & Cie GmbH.), January 11, 1988 (11. 01. 88), Full descriptions & EP, 249736, A & DE, 3620674, A & US, 4784994, A & DK, 8702548, A & PT, 84899, A | 1 - 11 |
| Y | JP, 63-174910, A (Shiseido Co., Ltd.), July 19, 1988 (19. 07. 88), Full descriptions | 1 - 11 |

Form PCT/ISA/210 (continuation of second sheet) (July 1992)